# EUROPEAN PATENT APPLICATION

(11) **EP 2 428 798 A1**
(43) Date of publication of application: **14.03.2012**
(21) Application number: 11178667.9
(22) Date of filing: 24.08.2011
(51) Int. Cl.: G01N 33/537, G01N 33/543, G01N 33/68, C12N 15/00, G01N 15/14, G01N 33/58, G01N 33/574

(54) **Fusion analyte cytometric bead assay, and systems and kits for performing the same**

(30) Priority: 14.09.2010 US 382805 P
(71) Applicant: Becton, Dickinson and Company, Franklin Lakes, NJ 07417 (US)
(72) Inventor: Wai, Hobert, San Francisco, CA California 94116 (US); Henriksson, Thomas Martin, Castro Valley, CA California 94546 (US); Warner, Brian David, Martinez, CA California 94553 (US)
(74) Representative: Korsten, Marius

(57) **Abstract**

Methods of detecting a fusion analyte in a sample are provided. Aspects of the methods include preparing a reaction mixture that includes a sample and a microparticle comprising a capture ligand for the fusion analyte, as well as first and second fluorescently labeled detector molecules. One of the first and second detector molecules specifically binds to the fusion analyte and the other specifically binds to a parent molecule thereof. Also provided are systems and kits configured for use in practicing methods of the invention.

## Description

### CROSS-REFERENCE TO RELATED APPLICATIONS

Pursuant to 35 U.S.C. § 119 (e) this application claims priority to the filing date of United States Provisional Patent Application Serial No. 61/382,805 filed September 14, 2010; the disclosure of which application is herein incorporated by reference.

### INTRODUCTION

Flow cytometers are well known analytical tools that enable the characterization of particles on the basis of light scatter and particle fluorescence. In a flow cytometer, particles are individually analyzed by exposing each particle to an excitation light, typically one or more lasers, and the light scattering and fluorescence properties of the particles are measured. Particles, such as molecules, analyte-bound beads, individual cells, or subcomponents thereof, typically are labeled with one or more spectrally distinct fluorescent dyes, and detection is carried out using a multiplicity of photodetectors, one for each distinct dye to be detected. Flow cytometers are commercially available from, for example, BD Biosciences (San Jose, CA).

Early in the development of flow cytometry, it was recognized that various types of ligand binding assays could be carried out using microparticles (beads) coated with one member of a binding pair. For example, immunoassays can be carried out in a sandwich hybridization assay format using beads coated with an analyte-specific binding agent, such as a monoclonal antibody (mAb), as a capture reagent, and a second analyte-specific binding agent, again typically a mAb, labeled with a fluorophore as a reporter reagent. The coated beads and reporters are incubated with a sample containing (or suspected of containing) the analyte of interest to allow for the formation of bead-analyte-reporter complexes. Analysis by flow cytometry enables both detecting the presence of bead-analyte-reporter complexes and simultaneously measuring the amount of reporter fluorescence associated with the complex as a quantitative measure of the analyte present in the sample.

It was also recognized early in the development of flow cytometry that the simultaneous analysis of multiple analytes in a sample could be carried out using a set of distinguishable beads, each type of bead coated with a unique analyte-specific binding agent. The bead set and fluorescently labeled reporter reagents, one for each species of analyte to be detected, are incubated with a sample containing the analytes of interest to allow for the formation of bead-analyte-reporter complexes for each analyte present, and the resulting complexes are analyzed by flow cytometry to identify and, optionally, quantify the analytes present in the sample. Because the identity of the analyte bound to the complex is indicated by the identity of the bead, multiple analytes can be simultaneously detected using the same fluorophore for all reporter reagents. A number of methods of making and using sets of distinguishable microparticles have been described in the literature.

UK Patent No. 1 561 042, published February 13, 1980, and Fulwyler and McHugh, 1990, Methods in Cell Biology 33:613-629, describe the use of multiple microparticles distinguished by size, wherein each size microparticle is coated with a different target-specific antibody.

Tripatzis, European Patent No. 0 126,450, published November 28, 1984 (see also corresponding Canadian Patent 1 248 873), describes multi-dimensional arrays of microparticles formed by labeling microparticles with two or more fluorescent dyes at varying concentrations. Microparticles in the array are uniquely identified by the levels of fluorescence dyes. Tripatzis describes the use of such arrays for the simultaneous detection a large numbers of analytes in a sample by flow cytometry, and, further, describes their use as labels in microscopy.

U.S. Patent Nos. 4,499,052 and 4,717,655, Entitled: "Method and Apparatus for Distinguishing Multiple Subpopulations of Cells", issued February 12, 1985, and January 5, 1988, respectively, describe the use of microparticles distinguishably labeled with two different dyes, wherein the microparticles are identified by separately measuring the fluorescence intensity of each of the dyes.

Both one-dimensional and two-dimensional arrays for the simultaneous analysis of multiple analytes by flow cytometry are available commercially. Examples of one-dimensional arrays of singly dyed beads distinguishable by the level of fluorescence intensity include the BD™ Cytometric Bead Array (CBA) (BD Biosciences, San Jose, CA) and Cyto-Plex™ Flow Cytometry microspheres (Duke Scientific, Palo Alto, CA). An example of a two-dimensional array of beads distinguishable by a combination of fluorescence intensity (five levels) and size (two sizes) is the QuantumPlex™ microspheres (Bangs Laboratories, Fisher, IN). An example of a two-dimensional array of doubly-dyed beads distinguishable by the levels of fluorescence of each of the two dyes is described in McDade and Fulton, April 1997, Medical Device & Diagnostic Industry; and Fulton et al., 1997, Clinical Chemistry 43(9):1749-1756.

In research and diagnostics, among other fields, the detection of fusion proteins in a sample finds use. Fusion proteins may arise from chromosomal aberrations such as translocations, inversions, insertions, deletions and other mutations within or among chromosomes. Many fusion proteins have been identified that are associated with disease conditions, such as cancer. Fusion proteins may be detected using a variety of different protocols, such as described in U.S. Patent No. 6,610,498. One protocol of interest is a sandwich based protocol, in which two different specific binding members that simultaneously bind to the fusion protein of interest are employed to detect the presence of the fusion protein. U.S. Patent No. 6,686,165 describes both "dipstick" and flow cytometric bead protocols for detecting the presence of a fusion protein, such as BCR-ABL, in a sample. The "BCR-ABL Protein Kit" from BD Biosciences, San Jose, CA, provides reagents for use in flow cytometric bead protocols for the detection of BCR-ABL in a sample.

### SUMMARY

Methods of detecting a fusion analyte in a sample are provided. Aspects of the methods include preparing a reaction mixture that includes a sample and a microparticle comprising a capture ligand for the fusion analyte, as well as first and second fluorescently labeled detector molecules. One of the first and second detector molecules specifically binds to the fusion analyte and the other specifically binds to a parent molecule thereof. Also provided are systems and kits configured for use in practicing methods of the invention.

### BRIEF DESCRIPTION OF THE FIGURES

FIG. 1 provides a schematic of a fusion protein detection method according to an embodiment of the invention.
FIG. 2 shows the correlation between BCR reactivity and BCR-ABL reactivity as determined in Example 2, below.

### DETAILED DESCRIPTION

Methods of detecting a fusion analyte in a sample are provided. Aspects of the methods include preparing a reaction mixture that includes a sample and a microparticle comprising a capture ligand for the fusion analyte, as well as first and second fluorescently labeled detector molecules. One of the first and second detector molecules specifically binds to the fusion analyte and the other specifically binds to a parent molecule thereof. Also provided are systems and kits configured for use in practicing methods of the invention.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments are specifically embraced by the present invention and are disclosed herein just as if each and every combination was individually and explicitly disclosed, to the extent that such combinations embrace operable processes and/or devices/systems/kits. In addition, all sub-combinations listed in the embodiments describing such variables are also specifically embraced by the present invention and are disclosed herein just as if each and every such sub-combination of chemical groups was individually and explicitly disclosed herein.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

In further describing embodiments of the invention, aspects of embodiments of the methods will be described first in greater detail. Next, embodiments of systems and kits that may be used in practicing methods of the invention are reviewed.

### METHODS

As summarized above, embodiments of the invention are directed to methods of detecting a fusion analyte in a sample. Fusion analytes that are detected in accordance with embodiments of the methods are molecules that include at least two distinct heterologous domains which are stably associated with each other. As these at least two distinct domains are heterologous, they are not wild-type molecules. A given molecule is a "wild-type" molecule if it is the typical form of the molecule as it occurs in nature, as distinguished from mutant forms. As such, a wild-type molecule is a molecule, e.g., nucleic acid or protein, which predominates in a natural population of organisms or strain of organisms, in contrast to that of natural or laboratory mutant forms of the molecule. Accordingly, fusion analytes are composed of at least two distinct domains of different origin. As the two domains of the fusion analytes are stably associated with each other, they do not dissociate from each other under cellular conditions, e.g., conditions at the surface of a cell, conditions inside of a cell, etc. The two domains may be associated with each other via covalent or non-covalent binding, as desired. In principle, the fusion analytes may be any type of molecule, and in some instances the fusion analytes are nucleic acids, proteins, or composites thereof, e.g., nucleic acid/peptide composite molecules.

In some instances, the fusion analytes are fusion proteins that include at least the two distinct domains described above. As such, fusion proteins that may be fusion analytes in methods described herein include at least a first domain and a second domain, where the two domains are heterologous. In some instances, the fusion protein includes a first domain which is found in a "parent" protein and a second heterologous domain which is found in a second protein that is distinct from the parent protein. Fusion proteins of interest include both recombinant fusion proteins, i.e., proteins that are expressed by man-made recombinant coding sequences, and chimeric mutant proteins, i.e., fusion proteins that are the product of a chromosomal rearrangement. The chromosomal rearrangement may be an intra-chromosomal rearrangement, such that two heterologous coding sequences on the same chromosome are fused together and encode the fusion protein. Examples of intra-chromosomal rearrangements include deletion rearrangements. The chromosomal rearrangement may also be an inter-chromosomal rearrangement, such that two heterologous coding sequences on non-homologous chromosomes are fused together and encode the fusion protein. As such, the fusion protein may arise from a translocation event. Fusion proteins of interest include, but are not limited to, those arising from the following translocations: 10q11; t(2;3)(q13); t(2;5)(p23;q35); t(8;14); t(8;21)(q22;q22); t(9;22)(q34;q11); t(11;14)(q13;q32);t(11;22)(q24;q11.2-12); t(12;21)(p12;q22); t(14;18)(q32;q21); t(17;22); t(15;17); t(1;12)(q21;p13); t(9;12)(p24;p13); t(X;18)(p11.2;q11.2); t(1;11)(q42.1;q14.3); and t(12;15)(p13;q25). Specific fusion proteins of interest include, but are not limited to: RET-PTC; PAX8-PPARγ1; c-myc-IGH; AML1-ETO; BCR-Abl fusion proteins, such as BCR-ABL1; TEL-AML1; Bcl-2; TEL-JAK2; gag-one fusion proteins; TRP-MET; TEL-PDGFRβ; FIP1L1-PDGFRα; E2A-PBX1; PML-RARAa; and ZNF198-FGFR1 etc.

As summarized above, embodiments of the methods are directed to detecting a fusion analyte in a sample. As such, aspects of the invention include determining whether a fusion analyte is present in a sample, e.g., determining the presence or absence of one or more fusion analytes in a sample. In certain embodiments of the methods, the presence of one or more fusion analytes in the sample may be determined qualitatively or quantitatively. Qualitative determination includes determinations in which a simple yes/no result with respect to the presence of a fusion analyte in the sample is provided to a user. Quantitative determination includes both semi-quantitative determinations in which a rough scale result, e.g., low, medium, high, is provided to a user regarding the amount of fusion analyte in the sample and fine scale results in which an exact measurement of the concentration of the fusion analyte is provided to the user.

In practicing methods of the invention, a variety of different types of samples may be assayed. Samples that may be assayed in the subject methods may vary, and include both simple and complex samples. Simple samples are samples that include the fusion analyte of interest, and may or may not include one or more molecular entities that are not of interest, where the number of these non-interest molecular entities may be low, e.g., 10 or less, 5 or less, etc. Simple samples may include initial biological or other samples that have been processed in some manner, e.g., to remove potentially interfering molecular entities from the sample, inhibit proteases, etc. By "complex sample" is meant a sample that may or may not have the analytes of interest, but also includes many different proteins and other molecules that are not of interest. In some instances, the complex sample assayed in the subject methods is one that includes 10 or more, such as 20 or more, including 100 or more, e.g., 10³ or more, 10⁴ or more (such as 15,000; 20,000 or 25,000 or more) distinct (i.e., different) molecular entities, that differ from each other in terms of molecular structure.

In practicing the subject methods, the first step is to provide a sample that is to be assayed for the presence of the fusion analyte of interest. The sample may be any of a variety of different types of samples, where the sample may be used directly from an initial source as is, e.g., where it is present in its initial source as a fluid, or preprocessed in some manner, e.g., to provide a fluid sample from an initial non-fluid source, e.g., solid or gas; to dilute and or concentrate an initial fluid sample, etc. Samples of interest include, but are not limited to: biological fluids, e.g., blood, tears, saliva; biological solids that are treated to produce fluid samples, e.g., tissues, organs, etc., which are subjected to one or more processing steps, e.g., homogenization, etc., to produce a fluid sample; etc. In certain embodiments, the samples of interest are biological samples, such as, but not limited to, urine, blood, serum, plasma, saliva, perspiration, feces, cheek swabs, cerebrospinal fluid, cell lysate samples, and the like. The sample can be a biological sample or can be extracted from a biological sample derived from humans, animals, plants, fungi, yeast, bacteria, tissue cultures, viral cultures, or combinations thereof using conventional methods for the successful extraction of DNA, RNA, proteins and peptides. In some instances, the sample is a cell lysate prepared from an initial cellular sample. In such instances, any suitable lysing protocol may be employed, e.g., contacting a cellular sample with a lysing agent and separating the resultant cellular debris from the supernatant to obtain a cell lysate. Many cell lysates contain proteases that must be inhibited to accurately detect an analyte of interest. Use of a protein, known to be present in the cell, as a control according to embodiments of the invention can be used to show adequacy of the protease inhibitor treatment in such assays.

Following obtainment of the sample and any desired preparation thereof, the sample is combined with microparticles and first and second detector molecules to produce a reaction mixture. As used herein, the terms "microparticles", "microbeads", or "beads" are used interchangeably. These terms refer to small particles with a diameter in the nanometer to micrometer range, such as from 0.01 to 1,000 µm in diameter, for example from 0.1 to 100 µm in diameter, and including from 1 to 100 µm in diameter, and, for use in flow cytometry, including from about 1 to 10 µm in diameter. Microparticles can be of any shape, and in some instances are approximately spherical ("microspheres"). Microparticles serve as solid supports or substrates to which other materials, such as capture ligands, can be coupled. Microparticles can be made of any appropriate material (or combinations thereof), including, but not limited to polymers such as polystyrene; polystyrene which contains other co-polymers such as divinylbenzene; polymethylmethacrylate (PMMA); polyvinyltoluene (PVT); copolymers such as styrene/butadiene, styrene/vinyltoluene; latex; or other materials, such as silica (e.g., SiO₂). Microparticles suitable for use in the present invention are well known in the art and commercially available from a number of sources. Unstained microspheres in a variety of sizes and polymer compositions that are suitable for the preparation of fluorescent microparticles of the invention are available from a variety of sources, including: Bangs Laboratories (Carmel, Ind.), Interfacial Dynamics Corporation (Portland, Oreg.), Dynal (Great Neck, N.Y.), Polysciences (Warrington, Pa.), Seradyne (Indianapolis, Ind.), Magsphere (Pasadena, Calif.), Duke Scientific Corporation (Palo Alto, Calif.), Spherotech Inc. (Libertyville, Ill.) and Rhone-Poulenc (Paris, France). Chemical monomers for preparation of microspheres are available from numerous sources.

Aspects of microparticles finding use in methods of the invention include the presence of a capture ligand that specifically binds to a common region of the first domain of the target fusion analyte and the parent molecule of the fusion analyte. As used herein, a molecule is a parent molecule of a given fusion analyte if a portion of the parent molecule is found in the fusion analyte. The capture ligand is therefore a ligand that binds to a region or epitope that is found on both the first domain of the fusion analyte that is derived from the parent molecule, as well as the parent molecule itself. As such, the capture ligand binds to an identical epitope found on both the fusion analyte and the parent molecule of the fusion analyte. In certain embodiments, the affinity between a capture ligand and fusion analyte first domain/ parent molecule to which it specifically binds when they are specifically bound to each other in a binding complex is characterized by a K_{D} (dissociation constant) of less than 10⁻⁶ M, less than 10⁻⁷ M, less than 10⁻⁸ M, less than 10⁻⁹ M, less than 10⁻¹⁰ M, less than 10⁻¹¹ M, less than 10⁻¹² M, less than 10⁻¹³ M, less than 10⁻¹⁴ M, or less than 10⁻¹⁵ M. A variety of different types of specific binding agents may be employed as the capture ligands. Specific binding agents of interest include antibody binding agents, proteins, peptides, haptens, nucleic acids, etc. The term "antibody binding agent" as used herein includes polyclonal or monoclonal antibodies or fragments that are sufficient to bind to an analyte of interest. The antibody fragments can be, for example, monomeric Fab fragments, monomeric Fab' fragments, or dimeric F(ab)'₂ fragments. Also within the scope of the term "antibody binding agent" are molecules produced by antibody engineering, such as single-chain antibody molecules (scFv) or humanized or chimeric antibodies produced from monoclonal antibodies by replacement of the constant regions of the heavy and light chains to produce chimeric antibodies or replacement of both the constant regions and the framework portions of the variable regions to produce humanized antibodies. The capture ligand may be stably associated with the surface of the microparticles using any convenient protocol, e.g., via covalent bonding with functional groups present on the surface of the microparticle.

The specific capture ligand employed will vary depending on the specific fusion analyte of interest. Examples of capture ligands for BCR-ABL that may be employed include, but are not limited to: moABs 7C6, ER-FP1, Yae, as described in U.S. Patent No. 6,686,165; and the like.

In some instances, the microparticles include one or more fluorescent dyes incorporated therein, i.e., they are stained with one or more fluorescent dyes. Fluorescent dyes have been incorporated into uniform microspheres in a variety of ways, for example by copolymerization of the fluorescent dye into the microspheres during manufacture (U.S. Pat. No. 4,609,689 to Schwartz et al. (1975), U.S. Pat. No. 4,326,008 to Rembaum (1982), both incorporated by reference); by entrapment of the fluorescent dye into the microspheres during the polymerization process; or by non-covalent incorporation of the fluorescent dye into previously prepared microspheres (U.S. Pat. Nos. 5,326,692; 5,723,218; 5,573,909; 5,786,219; and 6,514,295; each incorporated by reference). The method of labeling the microspheres is not a critical aspect of the invention; any method that allows the labeling of the microparticles with a controllable amount of dye can be used.

Fluorescent dyes (fluorophores) can be selected from any of the many dyes suitable for use in imaging applications (e.g., flow cytometry). A large number of dyes are commercially available from a variety of sources, such as, for example, Molecular Probes (Eugene, OR) and Exciton (Dayton, OH). Examples of fluorophores that may be incorporated into the microparticles include, but are not limited to, 4-acetamido-4'-isothiocyanatostilbene-2,2'disulfonic acid; acridine and derivatives such as acridine, acridine orange, acrindine yellow, acridine red, and acridine isothiocyanate; 5-(2'-aminoethyl)aminonaphthalene-1-sulfonic acid (EDANS); 4-amino-N-[3-vinylsulfonyl)phenyl]naphthalimide-3,5 disulfonate (Lucifer Yellow VS); N-(4-anilino-1-naphthyl)maleimide; anthranilamide; Brilliant Yellow; coumarin and derivatives such as coumarin, 7-amino-4-methylcoumarin (AMC, Coumarin 120), 7-amino-4-trifluoromethylcouluarin (Coumaran 151); cyanine and derivatives such as cyanosine, Cy3, Cy5, Cy5.5, and Cy7; 4',6-diaminidino-2-phenylindole (DAPI); 5', 5"-dibromopyrogallol-sulfonephthalein (Bromopyrogallol Red); 7-diethylamino-3-(4'-isothiocyanatophenyl)-4-methylcoumarin; diethylaminocoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein isothiocyanate (FITC), fluorescein chlorotriazinyl, naphthofluorescein, and QFITC (XRITC); fluorescamine; IR144; IR1446; Green Fluorescent Protein (GFP); Reef Coral Fluorescent Protein (RCFP); Lissamine™; Lissamine rhodamine, Lucifer yellow; Malachite Green isothiocyanate; 4-methylumbelliferone; ortho cresolphthalein; nitrotyrosine; pararosaniline; Nile Red; Oregon Green; Phenol Red; B-phycoerythrin; o-phthaldialdehyde; pyrene and derivatives such as pyrene, pyrene butyrate and succinimidyl 1-pyrene butyrate; Reactive Red 4 (Cibacron™ Brilliant Red 3B-A); rhodamine and derivatives such as 6-carboxy-X-rhodamine (ROX), 6-carboxyrhodamine (R6G), 4,7-dichlororhodamine lissamine, rhodamine B sulfonyl chloride, rhodamine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'-tetramethyl-6-carboxyrhodamine (TAMRA), tetramethyl rhodamine, and tetramethyl rhodamine isothiocyanate (TRITC); riboflavin; rosolic acid and terbium chelate derivatives; xanthene; or combinations thereof. Other fluorophores or combinations thereof known to those skilled in the art may also be used, for example those available from Molecular Probes (Eugene, OR) and Exciton (Dayton, OH).

In addition to the microspheres, the sample is also combined with first and second detector molecules in order to produce a reaction mixture. The first and second detector molecules each include a specific binding domain and a label domain. In the first detector molecule, the specific binding domain specifically binds to the second heterologous domain of the fusion analyte. As such, both the first detector molecule and the capture ligand may bind simultaneously to the fusion analyte, such that a binding complex is produced which is made up of the capture ligand, fusion analyte and first detector molecule. The specific binding domain may be any convenient binding domain, e.g., a binding domain as reviewed above in discussion of the capture ligand. Examples of specific binding domains for BCR-ABL include, but are not limited to: moABs 8E9, G98-271.1.3 as described in U.S. Patent No. 6,686,165, and the like. With respect to the label, in some instances the label is a first fluorescent label which produces a first fluorescent signal upon excitation. Suitable fluorescent labels include, but are not limited to, those described above in connection with the microparticles.

The specific binding domain of the second detector molecule specifically binds to a second region of the parent molecule that is different from the region, e.g., epitope, which is bound by the capture ligand. As such, both the second detector molecule and capture ligand may bind simultaneously to the parent molecule, such that a binding complex is produced that is made up of the capture ligand, parent molecule and second detector molecule. The specific binding domain may be any convenient binding domain, e.g., a binding domain as reviewed above in discussion of the capture ligand. With respect to the label, in some instances the label is a second fluorescent label which produces a second fluorescent signal that is distinguishable from the first fluorescent signal.

Accordingly, the first and second fluorescent signals produced upon excitation of the first and second fluorescent labels are distinguishable from each other, meaning that both can be detected at the same time and that the signal from one does not modify or change the signal from the other. In addition, if the microparticles are labeled with one or more fluorescent labels, e.g., as described above, the first and second fluorescent labels produce signals that are also distinguishable from any microparticle fluorescent labels. For example, the microparticles may be stained with a third fluorescent label which produces a third fluorescent signal that is distinguishable from the first and second fluorescent signals.

In preparing the reaction mixture, the sample may be combined with these reaction components, i.e., the microparticles and first and second detectors, using any convenient protocol. The microparticles and detector molecules may be simultaneously combined with the sample, or one or more components sequentially contacted with the sample. Combination may be carried out with mixing, as desired.

Contact of the sample with the reaction components is performed under incubation conditions that provide for binding of capture ligands and detector molecules to their respective parent molecules and fusion analytes, if present, in the sample. In some instances, the reaction components and samples are contacted and combined at a temperature ranging from 15 to 50, such as from 20 to about 40 °C. Contact may be performed with mixing or agitation, e.g., with vortexing etc., to provide for sufficient combination of the reaction components and the sample. The resultant reaction mixture may then be maintained or incubated for a period of time prior to flow cytometric analysis. In some instances, the reaction mixture is incubated at a temperature ranging from 15 to 50, such as from 20 to about 40 °C for a period of time ranging from about 30 minutes to 72 hours, such as 1 hour to 24 hours, including 1 hour to 3 hours. Following the above incubation step, the sample may be assayed immediately or stored for assay at a later time. If stored, in some embodiments the sample is stored at a reduced temperature; e.g., on ice.

Where desired, the microparticles in the resultant reaction mixture may be washed, e.g., to remove any unbound detector molecules and other sample components. Washing may be performed using any convenient protocol, e.g., by combining the reaction mixture with a suitable wash buffer and separating the microparticles from the fluid. A given washing protocol may include one or more distinct washing steps, as desired. Following any washing protocol, the microparticles may be re-suspended in a suitable liquid, e.g., the washing buffer or another buffer, for flow cytometric analysis.

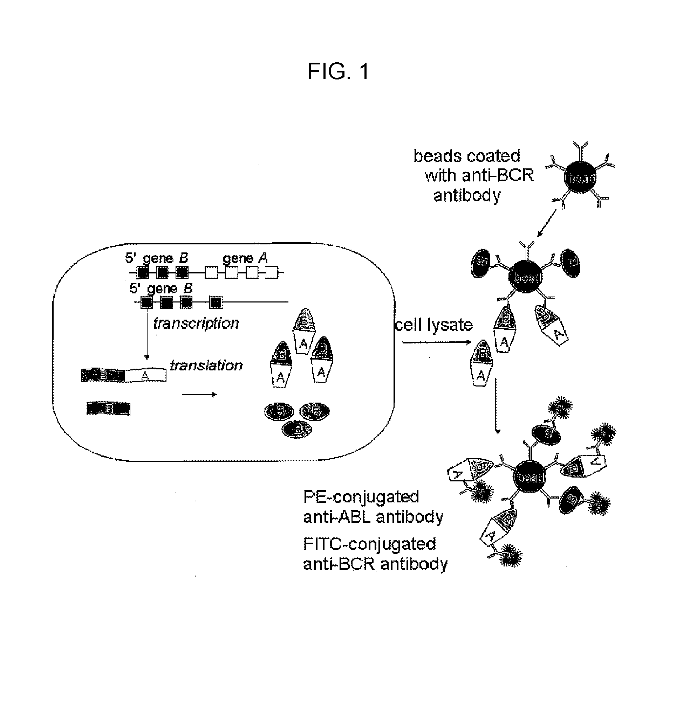

An embodiment of the above microparticle preparation protocol is depicted schematically in FIG. 1. In FIG. 1, the cell depicted in the figure comprises coding sequences for both a parent protein (gene B) and a fusion protein (gene B-gene A). Transcription of these two coding sequences produces both fusion protein (AB) and parent protein (B). A lysate of the cell is prepared and combined with microparticles coated with a capture antibody that specifically binds to (B). The lysate is also combined with PE-conjugated anti (A) antibody and FITC-conjugated anti-B antibody. As shown in the figure, combination results in production of microparticles having surface binding complexes of: (a) capture antibody-AB fusion protein- PE-conjugated anti (A) antibody; and (b) capture antibody-B parent protein- FITC-conjugated anti (B) antibody. The resultant microparticles are ready for flow cytometric analysis.

Following preparation of the microparticles, e.g., as described above, the microparticles from the reaction mixture are flow cytometrically assayed for the first and second fluorescent signals to detect the presence of the fusion analyte in the sample. Flow cytometry is a well-known methodology using multi-parameter data for identifying and distinguishing between different particle types (i.e., particles that vary from one another terms of label (wavelength, intensity), size, etc., in a fluid medium. In flow cytometrically analyzing the microparticles prepared as described above, a liquid medium comprising the microparticles is first introduced into the flow path of the flow cytometer. When in the flow path, the microparticles are passed substantially one at a time through one or more sensing regions, where each of the microparticles is exposed separately individually to a source of light at a single wavelength and measurements of light scatter parameters and/or fluorescent emissions as desired (e.g., two or more light scatter parameters and measurements of one or more fluorescent emissions) are separately recorded for each microparticle. The data recorded for each microparticle is analyzed in real time or stored in a data storage and analysis means, such as a computer, as desired. U.S. Pat. No. 4,284,412 describes the configuration and use of a typical flow cytometer equipped with a single light source while U.S. Pat. No. 4,727,020 describes the configuration and use of a flow cytometer equipped with two light sources. The disclosures of these patents are herein incorporated by reference. Flow cytometers having more than two light sources may also be employed.

More specifically, in a flow cytometer, the microparticles are passed, in suspension, substantially one at a time in a flow path through one or more sensing regions where in each region each microparticle is illuminated by an energy source. The energy source may include an illuminator that emits light of a single wavelength, such as that provided by a laser (e.g., He/Ne or argon) or a mercury arc lamp with appropriate filters. For example, light at 488 nm may be used as a wavelength of emission in a flow cytometer having a single sensing region. For flow cytometers that emit light at two distinct wavelengths, additional wavelengths of emission light may be employed, where specific wavelengths of interest include, but are not limited to: 535 nm, 635 nm, and the like.

In series with a sensing region, detectors, e.g., light collectors, such as photomultiplier tubes (or "PMT"), are used to record light that passes through each microparticle (generally referred to as forward light scatter), light that is reflected orthogonal to the direction of the flow of the microparticles through the sensing region (generally referred to as orthogonal or side light scatter) and fluorescent light emitted from the microparticles, if it is labeled with fluorescent marker(s), as the microparticle passes through the sensing region and is illuminated by the energy source. Each of forward light scatter (or FSC), orthogonal light scatter (SSC), and fluorescence emissions (FL1, FL2, etc.) comprise a separate parameter for each microparticle (or each "event"). Thus, for example, two, three or four parameters can be collected (and recorded) from a microparticle labeled with two different fluorescence markers.

Accordingly, in flow cytometrically assaying the particles, the microparticles which may or may not include fusion analyte and/or parent molecule binding complexes on their surface are detected and uniquely identified by exposing the microparticles to excitation light and measuring the fluorescence of each microparticle in one or more detection channels, as desired. The excitation light may be from one or more light sources and may be either narrow or broadband. Examples of excitation light sources include lasers, light emitting diodes, and arc lamps. Fluorescence emitted in detection channels used to identify the microparticles and binding complexes associated therewith may be measured following excitation with a single light source, or may be measured separately following excitation with distinct light sources. If separate excitation light sources are used to excite the microparticle labels, the labels may be selected such that all the labels are excitable by each of the excitation light sources used.

For example, a BD FACSCalibur dual laser flow cytometer (BD Bioscience, San Jose, CA) has 488 nm and 635 nm excitation lasers that are focused on the flow stream at spatially discrete regions, and detection optics designed to measure light in three detection channels, designated FL1, FL2, and FL3, following excitation by the 488 nm laser, and a fourth detection channel, designated FL4, following excitation by the 635 nm laser. The different detection channels are used to identify the signal from the microparticles and detector labels, as desired, where choice of a given detection channel for a given reaction component will depend on the particle label associated with the given reaction component.

Flow cytometers further include data acquisition, analysis and recording means, such as a computer, wherein multiple data channels record data from each detector for the light scatter and fluorescence emitted by each microparticle as it passes through the sensing region. The purpose of the analysis system is to classify and count microparticles wherein each microparticle presents itself as a set of digitized parameter values. In flow cytometrically assaying microparticles in methods of the invention, the flow cytometer may be set to trigger on a selected parameter in order to distinguish the microparticles of interest from background and noise. "Trigger" refers to a preset threshold for detection of a parameter. It is typically used as a means for detecting passage of microparticle through the laser beam. Detection of an event which exceeds the threshold for the selected parameter triggers acquisition of light scatter and fluorescence data for the particle. Data is not acquired for microparticles or other components in the medium being assayed which cause a response below the threshold. The trigger parameter may be the detection of forward scattered light caused by passage of a microparticle through the light beam. The flow cytometer then detects and collects the light scatter and fluorescence data for microparticle.

A particular subpopulation of interest is then further analyzed by "gating" based on the data collected for the entire population. To select an appropriate gate, the data is plotted so as to obtain the best separation of subpopulations possible. This procedure is typically done by plotting forward light scatter (FSC) vs. side (i.e., orthogonal) light scatter (SSC) on a two dimensional dot plot. The flow cytometer operator then selects the desired subpopulation of microparticles (i.e., those cells within the gate) and excludes microparticles which are not within the gate. Where desired, the operator may select the gate by drawing a line around the desired subpopulation using a cursor on a computer screen. Only those microparticles within the gate are then further analyzed by plotting the other parameters for these microparticles, such as fluorescence.

Flow cytometric analysis of the microparticles, as described above, yields qualitative and quantitative information about the presence of the fusion analyte and parent molecule in the sample being assayed. Where desired, the above analysis yields counts of the fusion analyte and parent molecule of interest in the sample. As such, the above flow cytometric analysis protocol provides data regarding the numbers of one or more different types of fusion analytes in a sample and one or more different corresponding parent molecules in a sample, where the parent molecules may serve as an internal control for the assay.

Aspects of the invention include the multiplex analysis of two or more distinct fusion analytes in a sample. By "multiplex analysis" is meant that two or more distinct fusion analytes and their corresponding parent molecules are analyzed, e.g., quantitatively. In some instances the number of sets of fusion analytes and parent molecules is greater than 2, such as 4 or more, 6 or more, 8 or more, etc., up to 20 or more, e.g., 50 or more. In such instances, cytometric bead arrays having sets of distinguishable microparticles may be employed. Both one-dimensional and two-dimensional arrays for the simultaneous analysis of multiple analytes by flow cytometry are available commercially. Examples of one-dimensional arrays of singly dyed beads distinguishable by the level of fluorescence intensity include the BD.TM. Cytometric Bead Array (CBA) (BD Biosciences, San Jose, Calif.) and Cyto-Plex™ Flow Cytometry microspheres (Duke Scientific, Palo Alto, Calif). An example of a two-dimensional array of beads distinguishable by a combination of fluorescence intensity (five levels) and size (two sizes) is the QuantumPlex™ microspheres (Bangs Laboratories, Fisher, Ind.). An example of a two-dimensional array of doubly-dyed beads distinguishable by the levels of fluorescence of each of the two dyes is described in McDade and Fulton, April 1997, Medical Device & Diagnostic Industry; and Fulton et al., 1997, Clinical Chemistry 43(9):1749-1756. Also of interest is the array system described in U.S. Patent No. 7,507,588, the disclosure of which is herein incorporated by reference.

Aspects of the invention also include methods in which two different fusion proteins with a common domain are detected, e.g., TEL-AML and AML-ETO. In such instances, an antibody to AML can be bound to a bead and the two fusion proteins distinguished with for example FITC-anti-TEL and PE-anti-ETO. These embodiments may or may not include a third detector molecule that is specific for the AML parent protein, e.g., as described above. As such, some embodiments do not include first and second detector molecules in which the second detector molecule specifically binds to a second region of a parent molecule and comprises a second fluorescent label which produces a second fluorescent signal that is distinguishable from the first fluorescent signal. Instead, these embodiments include first and second distinguishably labeled detector molecules that specifically bind to different first and second fusion proteins, where the first and second fusion proteins include a common domain.

Signals obtained from the detector channels for the first and second fluorescent labels are employed to determine the presence or absence of the fusion analyte and parent molecule in the sample. In methods of the invention, results with respect to the parent molecule are employed as an internal control for the given assay being performed. As such, the results obtained for the parent molecule may be employed to determine assay integrity, e.g., to determine whether the result obtained for the fusion analyte is free from defect, e.g., defect that may arise from product use, sample handling, etc. In this manner, the parent molecule serves as a "housekeeping" control, where detection of the presence of the parent molecule confirms that the assay was performed correctly and imparts confidence to the result obtained for the fusion analyte.

### DEVICES AND SYSTEMS

Aspects of the invention further include systems for use in practicing the subject methods. Systems of the invention may a flow cytometer configured to assay microparticles to obtain data and a signal processing module configured to detect the presence of the fusion analyte and a corresponding parent molecule in a sample from the obtained data. Flow cytometers of interest include, but are not limited, to those devices described in U.S. Patent Nos.: 4,704,891; 4,727,029; 4,745,285; 4,867,908; 5,342,790; 5,620,842; 5,627,037; 5,701,012; 5,895,922; and 6,287,791; the disclosures of which are herein incorporated by reference. In some instances, the flow cytometer includes: a flow channel; at least a first light source configured to direct light to an assay region of the flow channel (where in some instances the cytometer includes two or more light sources); a first detector configured to receive light of a first wavelength from the assay region of the flow channel; and a second detector configured to receive light of a second wavelength from the assay region of the flow channel.

Aspects of the invention further include signal processing module configured to receive signals from the first and second detectors and output a result of whether a fusion analyte and its parent molecule are present in a sample. These signal processing module may be integrated into the cytometer as a single device, or distributed from the cytometer where the signal processing module and cytometer are in communication with each other, e.g., via a wired or wireless communication protocol.

Accordingly, aspects of the invention further include systems, e.g., computer based systems, which are configured to detect the presence of a fusion analyte and parent molecule in sample, e.g., as described above. A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" references any hardware and/or software combination that will perform the functions required of it. For example, any processor herein may be a programmable digital microprocessor such as available in the form of a electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

Embodiments of the subject systems include the following components: (a) a communications module for facilitating information transfer between the system and one or more users, e.g., via a user computer, as described below; and (b) a processing module for performing one or more tasks involved in the quantitative analysis methods of the invention.

In certain embodiments, a computer program product is described comprising a computer usable medium having control logic (computer software program, including program code) stored therein. The control logic, when executed by the processor of the computer, causes the processor to perform functions described herein. In other embodiments, some functions are implemented primarily in hardware using, for example, a hardware state machine. Implementation of the hardware state machine so as to perform the functions described herein may be accomplished using any convenient method and techniques.

In addition to the sensor device and signal processing module, e.g., as described above, systems of the invention may include a number of additional components, such as data output devices, e.g., monitors and/or speakers, data input devices, e.g., interface ports, keyboards, etc., fluid handling components, power sources, etc.

In some instances, the systems may further include a reaction mixture derivative thereof (e.g., washed microparticles produced therefrom), where the reaction mixture is prepared as described above, e.g., by combining a sample; microparticles comprising a capture ligand that specifically binds to a common region of the first domain the parent molecule; a first detector molecule that specifically binds to the second heterologous domain and comprises a first fluorescent label which produces a first fluorescent signal upon excitation; and a second detector molecule that specifically binds to a second region of the parent molecule and comprises a second fluorescent label which produces a second fluorescent signal that is distinguishable from the first fluorescent signal.

### UTILITY

The subject methods and systems find use in a variety of different applications where detection, e.g., either qualitative or quantitative determination, of the presence of a fusion analyte in a sample is desired. Such applications include both research and diagnostic applications, e.g., applications where a subject is diagnosed with respect to a given condition, e.g., a disease condition.

### COMPUTER RELATED EMBODIMENTS

Aspects of the invention further include a variety of computer-related embodiments. Specifically, the data analysis methods described in the previous sections may be performed using a computer. Accordingly, the invention provides a computer-based system for analyzing data produced using the above methods in order to provide quantitative determination of a binding kinetic parameter of a binding interaction of interest.

In certain embodiments, the methods are coded onto a physical computer-readable medium in the form of "programming", where the term "computer readable medium" as used herein refers to any storage or transmission medium that participates in providing instructions and/or data to a computer for execution and/or processing. Examples of storage media include floppy disks, magnetic tape, CD-ROM, a hard disk drive, a ROM or integrated circuit, a magneto-optical disk, or a computer readable card such as a PCMCIA card and the like, whether or not such devices are internal or external to the computer. A file containing information may be "stored" on computer readable medium, where "storing" means recording information such that it is accessible and retrievable at a later date by a computer. Of interest as media are non-transitory media, i.e., physical media in which the programming is associate with, such as recorded onto, a physical structure. Non-transitory media does not include electronic signals in transit via a wireless protocol.

With respect to computer readable media, "permanent memory" refers to memory that is permanent. Permanent memory is not erased by termination of the electrical supply to a computer or processor. Computer hard-drive, CD-ROM, floppy disk and DVD are all examples of permanent memory. Random Access Memory (RAM) is an example of non-permanent memory. A file in permanent memory may be editable and re-writable.

### KITS

In yet another aspect, the present invention provides kits for practicing the subject methods, e.g., as described above. The subject kits may include microparticles comprising a capture ligand that specifically binds to a common region of the first domain the parent molecule; a first detector molecule that specifically binds to the second heterologous domain and comprises a first fluorescent label which produces a first fluorescent signal upon excitation; and a second detector molecule that specifically binds to a second region of the parent molecule and comprises a second fluorescent label which produces a second fluorescent signal that is distinguishable from the first fluorescent signal. In addition, the kits may include calibration beads, as described above. In addition, the kit may include one or more additional compositions that are employed, including but not limited to: buffers, diluents, cell lysing agents, etc., which may be employed in a given assay. Kits may further include control reagents, e.g., purified preparations of the fusion analyte (or analytes) and/or corresponding parent molecule(s) to be detected using the subject kits. The above components may be present in separate containers or one or more components may be combined into a single container, e.g., a glass or plastic vial.

In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

The following examples are offered by way of illustration and not by way of limitation.

### EXPERIMENTAL

### Example 1:

In the following example BCR was used as the housekeeping protein to control for degradation of the fusion protein BCR-ABL.

For this example test cell suspensions were made by adding 10% K562 cells to white blood cell preparation from two different donors. The white blood cell preparations were also without addition. Each test cell suspension in PBS with 5% fetal bovine serum (PBS/FBS) was split into two equal parts. The cell concentrations were adjusted to 10 × 10⁶ cells/ml with PBS/FBS. To one of the parts from each suspension was added 20 µl each of Pretreatment A and B (Part of the BASV assay kit, Cat # 643939, obtained from BD Biosciences) for each ml of cell suspension. To the other part no such addition was made. The cell suspensions were incubated on ice for 10 min. After this incubation period, the cells were spun down, the supernatant was removed, the cells were re-suspended in PBS/FBS and then spun down again. All supernatant was removed.

The cells were now lysed at a concentration of 25 × 10⁶ cells/ml. The lysis buffer was Cell Lysis Buffer (part of Fusion Protein Buffer Kit, Cat # 560272, from BD Biosciences). For samples that had undergone Pretreatment A and B in the previous step, the lysis buffer was supplemented with 1 × Lysate Treatment Reagent (part of the Fusion Protein Buffer Kit). For the other samples no such addition was used. The samples were lysed for 10 minutes on ice, followed by centrifugation for 10 minutes at 12000 rpm in a microcentrifuge. The supernatant was collected and stored at -80°C until testing.

The samples were tested as follows. A detection reagent was made of 50 µl/per test αBCR-C6 Beads, 50 µl/per test αABL-Phycoerythrin (both components of the above mentioned BASV kit), and 100 ng/test of fluorescein labeled monoclonal antibody 11C8 (directed against the C-terminal end of BCR). 100 µl of this reagent was mixed with 50 µl sample and incubated for 2hours on a plate shaker. The beads were collected, washed once with CBA Wash Buffer, and resuspended in 200 µl CBA Wash Buffer. The beads were read in a FACSCanto II flow cytometer using settings described in the BASV kit. 300 events were collected per test. Median Fluorescent Intensity (MFI) was determined for the Fluorescein (BCR) and Phycoerythrin (BCR-ABL) channels. Results can be seen in the following table.

| Sample | BCR Reactivity | | BCR-ABL Reactivity | |
|---|---|---|---|---|
| | No Protease Inhibitor | + Protease Inhibitor | No Protease Inhibitor | + Protease Inhibitor |
| Blank | 61 | | 47 | |
| 1890 | 61 | 265 | 46 | 49 |
| 1890+K562 | 56 | 781 | 50 | 1718 |
| 5180 | 60 | 535 | 49 | 48 |
| 5180+K562 | 61 | 989 | 50 | 1804 |

Without protection of protease inhibitors neither BCR nor BCR-ABL show any reactivity. With protease inhibitors present both BCR and BCR-ABL are protected. Furthermore, BCR-ABL only shows reactivity when K562 cells are present.

### Example 2

In the following example BCR was used as the housekeeping protein to control for degradation of the fusion protein BCR-ABL.

For this example test suspensions were prepared by adding 10% K562 cells to white blood cell suspensions from two different donors. The cell suspension were split into multiple parts and treated as in Example 1 with the following exceptions.

In the pretreatment step multiple combinations of the following protease inhibitors were tested: 20 mM, 10 mM, 5 mM, and 0 mM amino ethyl benzoyl fluoride (AEBSF, Pefabloc SC, Fluka, Cat # 76307), 1 mM and 0 mM phenyl methyl Sulfonyl fluoride (PMSF, Sigma, Cat # P7626), 1X or 0X Complete Protease Inhibitor Cocktail (Roche, Cat # 11873580001). In the lysis step the following supplements to the lysis buffer was used: 1X BaculoGold Protease Inhibitor Cocktail, 1X Complete Protease Inhibitor Cocktail, or no supplement.

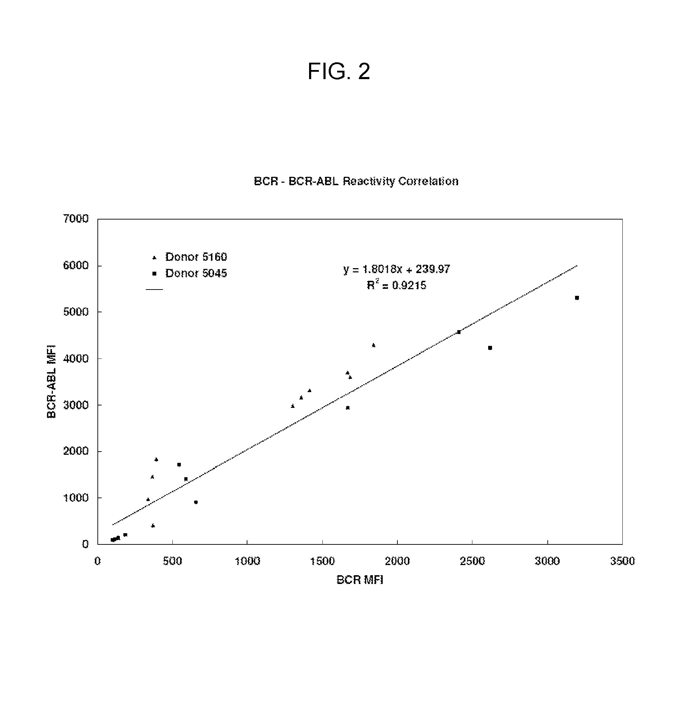

Each condition was tested as in Example 1. The correlation between BCR reactivity and BCR-ABL reactivity was determined and is shown in Figure 2. The blue rectangle in the lower left corner shows the area below the negative cutoff for both channels. There is a strong correlation between BCR and BCR-ABL reactivities. BCR reactivity is a good predictor of the success or failure of the protease inhibitor treatment.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

Accordingly, the preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope and spirit of present invention is embodied by the appended claims.

## Claims

1. A method of detecting a fusion analyte in a sample, wherein the fusion analyte comprises a first domain from a parent molecule and a second heterologous domain, the method comprising:
(a) combining a sample with:
(i) microparticles comprising a capture ligand that specifically binds to a common region of the first domain and the parent molecule;
(ii) a first detector molecule that specifically binds to the second heterologous domain and comprises a first fluorescent label which produces a first fluorescent signal upon excitation; and
(iii) a second detector molecule that specifically binds to a second region of the parent molecule and comprises a second fluorescent label which produces a second fluorescent signal that is distinguishable from the first fluorescent signal;
to produce a reaction mixture; and
(b) flow cytometrically assaying microparticles from the reaction mixture for the first and second fluorescent signals to detect the presence of the fusion analyte in the sample.

2. The method according to Claim 1, wherein the fusion analyte is a fusion protein.

3. The method according to Claim 2, wherein the fusion protein is the product of a chromosomal rearrangement.

4. The method according to Claim 3, wherein the chromosomal rearrangement is an inter-chromosomal rearrangement.

5. The method according to Claim 4, wherein the chromosomal rearrangement is an intra-chromosomal rearrangement.

6. The method according to Claim 1, wherein the method quantitatively detects the fusion analyte in the sample.

7. The method according to Claim 1, wherein the sample is a cell lysate.

8. The method according to Claim 1, wherein the first and second detector molecules are fluorescently labeled antibodies or binding fragments thereof.

9. The method according to Claim 1, wherein the microparticles are stained with a third fluorescent label which produces a third fluorescent signal that is distinguishable from the first and second fluorescent signals.

10. The method according to Claim 1, wherein the flow cytometrically assaying comprises exposing the reaction mixture to a single wavelength of light.

11. The method according to Claim 1, wherein the flow cytometrically assaying comprises exposing the reaction mixture to first and second wavelengths of light.

12. A flow cytometric system comprising:
a flow channel;
a first light source configured to direct light to an assay region of the flow channel;
a first detector configured to receive light of a first wavelength from the assay region of the flow channel;
a second detector configured to receive light of a second wavelength from the assay region of the flow channel; and
a signal processing module configured to receive signals from the first and second detectors and output a result of whether a fusion analyte and its parent molecule are present in a sample.

13. The flow cytometric system according to Claim 12, wherein the system further comprises a reaction mixture present in the flow channel, wherein the reaction mixture comprises:
(a) a sample;
(b) microparticles comprising a capture ligand that specifically binds to a common region of the first domain and the parent molecule;
(c) a first detector molecule that specifically binds to the second heterologous domain and comprises a first fluorescent label which produces a first fluorescent signal upon excitation; and
(d) a second detector molecule that specifically binds to a second region of the parent molecule and comprises a second fluorescent label which produces a second fluorescent signal that is distinguishable from the first fluorescent signal.

14. A kit for use in detecting a fusion analyte in a sample, wherein the fusion analyte comprises a first domain from a parent molecule and a second heterologous domain, the kit comprising:
(a) microparticles comprising a capture ligand that specifically binds to a common region of the first domain and the parent molecule;
(b) a first detector molecule that specifically binds to the second heterologous domain and comprises a first fluorescent label which produces a first fluorescent signal upon excitation; and
(c) a second detector molecule that specifically binds to a second region of the parent molecule and comprises a second fluorescent label which produces a second fluorescent signal that is distinguishable from the first fluorescent signal.

15. The kit according to Claim 14, wherein the first and second detector molecules are fluorescently labeled antibodies or binding fragments thereof and the microparticles are stained with a third fluorescent label which produces a third fluorescent signal that is distinguishable from the first and second fluorescent signals.
